# FASCICULE DE BREVET EUROPEEN

(11) **EP 0 493 151 B1**
(45) Date de publication et mention de la délivrance du brevet: **02.08.1995**
(21) Numéro de dépôt: 91403225.5
(22) Date de dépôt: 28.11.1991
(51) Int. Cl.: A61K 35/78, A61K 45/06, A61K 7/48

(54) **Composition amincissante à base de ginkgo biloba en tant qu'alpha-2 bloqueurs**
Schlankmachende Zusammensetzung mit Ginko Biloba als alpha-2-Blocker
Slimming composition containing ginkgo biloba as alpha-2 blocker

(30) Priorité: 28.11.1990 FR 9014864
(43) Date de publication de la demande: 01.07.1992
(73) Titulaire: L'OREAL, 75008 Paris (FR)
(72) Inventeur: Soudant, Etienne, F-94260 Fresnes (FR); Nadaud, Jean-François, F-75013 Paris (FR)
(74) Mandataire: Stalla-Bourdillon, Bernard

(56) Documents cités:
- EP-A- 0 120 165

## Description

La présente invention a pour objet l'utilisation du ginkgo biloba en tant qu'alpha-2-bloqueur en association avec au moins un autre alpha-2-bloqueur dans la préparation d'une composition amincissante destinée à une application topique ainsi que de telles compositions.

On sait que le gonflement du tissu conjonctif sous-cutané appelé cellulite donne à la peau un aspect "capitonné". La cellulite est constituée par l'accumulation locale de graisse et d'eau emprisonnées dans une gangue à compartiments plus ou moins étanches.

L'application topique d'un agent anti-cellulitique est susceptible d'enrayer l'accumulation locale de graisse par une action lipolytique et ainsi d'améliorer l'aspect esthétique de la peau.

Parmi les méthodes de stimulation de la lipolyse, la plus connue et la plus utilisée est celle qui consiste à inhiber la phosphodiestérase afin d'empêcher ou du moins limiter la vitesse de dégradation de l'AMP cyclique. En effet, la phosphodiestérase détruit l'AMP cyclique en le transformant en 5'AMP de telle sorte qu'il ne peut jouer le rôle d'activateur de la lipolyse. L'inhibition de l'action de la phosphodiestérase augmente ainsi le taux d'AMP cyclique au niveau des adipocytes ce qui stimule l'activité lipolytique.

Parmi les différents inhibiteurs de phosphodiestérase qui ont été préconisés comme amaigrissant, on peut en particulier mentionner les bases xanthiques et plus particulièrement la caféine.

Toutefois, il existe d'autres méthodes de lutte contre l'accumulation locale de graisse telles que le bloquage des récepteurs alpha-2 à la surface des adipocytes par des produits dits alpha-2-bloqueurs.

Les récepteurs alpha-2 sont normalement stimulés par les produits naturels du corps (les catécholamines) afin d'augmenter la teneur lipidique de ces cellules par exemple en périodes de diète ou de stress. En bloquant cette action, les alpha-2-bloqueurs empêchent ainsi l'accumulation de graisses dans les tissus adipeux et favorisent en même temps la libération des graisses.

Les alpha-2-bloqueurs connus (par exemple la yohimbine) ont jusqu'à présent été utilisés par voie orale dans différents buts dont celui de traiter l'obésité. D'autre part, GREENWAY et coll. (Clinical Therapeutics, 1987, vol 9 n° 6 p.663 à 669) ont montré que des crèmes à base de yohimbine, appliquées sur des femmes par ailleurs soumises à de fortes restrictions caloriques et à de l'exercice physique, avaient un effet lipolytique indirect. Mais la yohimbine et les alpha-2-bloqueurs de ce type pénètrent mal (GREENWAY et coll. sont obligés d'utiliser des enveloppements chauds) et ils ont des effets secondaires qui prohibent leur utilisation en cosmétique.

Il a maintenant été découvert de manière tout à fait inattendue que certains produits déjà connus en cosmétique avaient une action alpha-2-bloquante quand ceux-ci étaient présents en concentration importante.

En effet, la demanderesse a constaté que ces alpha-2-bloqueurs n'agissent qu'au delà d'une certaine concentration. C'est pour cette raison qu'un certain nombre de produits déjà connus en cosmétique, bien qu'ils aient peut-être un potentiel alpha-2-bloqueur, n'ont pas été reconnus jusqu'à maintenant comme ayant cette caractéristique.

Parmi les produits ayant cette action alpha-2-bloquante à haute concentration, on peut citer notamment le ginkgo biloba, le lierre et l'escine.

On connaît déjà l'utilisation du ginkgo biloba en cosmétique notamment pour son action vasodilatatrice ce qui augmente le calibre des vaisseaux (habituellement sanguins) par relâchement de la musculature.

A des concentrations modérées, les vasodilatateurs tels que le ginkgo biloba sont employés dans les compositions cosmétiques par exemple pour maintenir une bonne teneur d'hydratation de la peau (demande de brevet européen n° 275.005).

Toutefois, le ginkgo biloba n'a jamais été décrit comme ayant une activité lipolytique.

Bien que la découverte de l'activité alpha-2-bloquante du ginkgo biloba puisse suggérer son utilité dans une composition amincissante, la demanderesse a constaté que lorsqu'il est le seul principe actif, les concentrations élevées de ce produit, nécessaires pour obtenir l'effet voulu, entrainaient un risque d'action vasodilatatrice trop important ce qui présente un inconvénient pour les compositions amincissantes ou anticellulitiques destinées à une application topique.

En outre, il existe pour ce produit des problèmes de formulation en composition cosmétique, car au delà d'un certain seuil, le ginkgo biloba apporte une odeur et une couleur rédibitoire et s'avère également difficilement solubilisable à haute concentration.

Il y avait donc un problème double d'une part de garder une haute concentration d'alpha-2-bloqueur dans la composition cosmétique afin de maintenir l'effet stimulateur de la lipolyse voulu et d'autre part de limiter la concentration en ginkgo biloba.

Parmi les autres produits pour lesquels la demanderesse a démontré une action alpha-2-bloquante à haute concentration, le lierre et l'escine sont également déjà connus en cosmétique et en particulier dans des compositions amincissantes.

Le lierre a été utilisé en association avec des extraits de reine des prés, de centella asiatica et de laminaire (brevet français n° 2.571.616), en association avec une hormone (brevet français n° 2.571.616) ou en association avec de l'extrait d'arnica et de l'extrait de noix de cola (brevet français n° 2.499.405) ou avec une base purique et du fragon (brevet français n° 2.554.344) ou bien en association avec une substance à action rubéfiante (brevet français n° 2.573.306).

L'escine est habituellement utilisée comme composé vasoactif dans des compositions amincissantes, en particulier en association avec des mucopolysaccharides dont il renforce et accélère l'activité amincissante sans pour autant agir directement sur l'adipocyte, tout en protégeant les vaisseaux sanguins (brevet français n° 2.463.617, brevet belge n° 888.136, brevet français n° 2.400.902 et brevet français n° 2.356.427).

Notamment, et comme pour le ginkgo biloba, ces deux produits ne sont employés en cosmétique qu'en fonction de leurs caractéristiques autres qu'alpha-2-bloquantes.

Bien que la découverte de l'activité alpha-2-bloquante du lierre ainsi que de l'escine, comme du ginkgo biloba, pouvait suggérer une utilité dans une composition amincissante comme seul principe actif, la demanderesse a constaté qu'à des concentrations élevées nécessaires pour obtenir l'effet voulu, l'escine avait un effet collant et le lierre posait des problèmes de couleur, s'agissant de deux inconvénients importants en cosmétique.

L'invention a donc pour objet l'utilisation du ginkgo biloba en tant qu'alpha-2-bloqueur, en association avec au moins un autre alpha-2-bloqueur dans la préparation d'une composition amincissante destinée à une application topique.

En effet, la demanderesse a constaté que les inconvénients liés à la formulation du ginkgo biloba à concentration élevée pouvaient être résolus ou réduits par la mise en association de ce produit avec un autre alpha-2-bloqueur, de préférence l'escine et/ou le lierre tout en gardant une concentration en alpha-2-bloqueur suffisante pour agir contre la cellulite, en formulation cosmétique.

En outre, puisque l'escine et le lierre manifestent également une activité vasoconstrictrice, l'association selon l'invention permet de corriger l'effet vasodilatateur indésirable du ginkgo biloba.

De plus, comme les solvants des trois produits précisés ci-dessus ne sont pas les mêmes, on peut donc mettre plus d'alpha-2-bloqueur dans une formule contenant deux substances qu'avec une seule.

Le solvant de préférence utilisé pour le ginkgo biloba est le diéthylène glycol monoéthyl éther, en particulier le produit vendu sous la dénomination "TRANSCUTOL" par la Société GATTEFOSSE. Pour le lierre et l'escine, on utilise de préférence l'eau comme solvant.

Par emploi de l'expression "ginkgo biloba" on entend les extraits de la plante du même nom. Selon l'invention, on utilise de préférence les extraits vendus sous la dénomination de "Extrait Standard de ginkgo biloba" par la Société BEAUFOUR, obtenu par extraction dans un mélange acétone/eau à partir des feuilles ou ceux vendus sous la dénomination d'"Extrait Sec de ginkgo biloba" vendu par la Société INVERNI.

Par emploi de l'expression "escine" on entend l'isomère α ainsi que ses sels notamment le sel de sodium, l'escine étant un extrait de Marron d'Inde.

Par emploi de l'expression "lierre" on doit entendre les extraits de Hedera haelix L. (ou lierre grimpant) contenant des saponines. Ces extraits peuvent être préparés par tout procédé permettant d'éliminer les constituants tels que protéines, lipides, glucides, mucilages et tanins. Selon l'invention, on préfère utiliser les extraits de lierre vendus sous la dénomination de "Lierre Grimpant Saponines Totales" par la Société INVERNI ou l'"Extrait de Lierre" vendu par la Société GATTEFOSSE.

L'association selon l'invention permet par ailleurs de se protéger contre l'effet négatif du stress. Ceci résulte du fait que le stress provoque une décharge d'épinéphrine (une catécholamine naturelle) ayant des effets engraissants. Il s'agit en particulier du niveau fémoral du corps de la femme, c'est-à-dire la "culotte de cheval" (P. MAURIEGE et coll. European Journal of Clinical Investigation (1987) vol 17, p.156 à 165).

Grâce à l'association selon l'invention, les substances libérées par le corps humain lors d'un stress et qui, sans elle, vont fortement inhiber la lipolyse au niveau de la zone fémorale, vont avoir l'effet inverse, c'est-à-dire vider les adipocytes de leur réserve graisseuse. Par ailleurs, dans ces zones, l'association selon l'invention a plus d'effet amaigrissant que le régime hypocalorique le plus drastique car les récepteurs alpha-2 ont pour but d'éviter dans ces conditions de régime, les mobilisations des graisses adipeuses.

L'association d'un régime de ce type avec une application topique de l'association d'alpha-2-bloqueurs selon l'invention est tout de même recommandée et synergique, car le régime seul ne fait pas diminuer la culotte de cheval. Une crème contenant des actifs lipolytiques non alpha-2-bloqueurs n'a que très peu d'effet dans cette zone. Par contre, une crème contenant des actifs lipolytiques dont des alpha-2-bloqueurs a des effets ; néanmoins ces effets sont nettement renforcés quand on associe le régime et l'application topique d'une composition à base d'alpha-2-bloqueurs car les acides gras libérés par la composition sont utilisés en tant que source d'énergie pour le corps. La même relation synergique existe entre le sport et l'application topique d'une crème à base d'alpha-2-bloqueurs.

La teneur totale en alpha-2-bloqueur dans la composition résultant de l'association selon l'invention est généralement supérieure à 0,03 % et de préférence comprise entre 0,05 et 20 % en poids.

D'une manière générale, les compositions cosmétiques amincissantes selon l'invention contiennent le ginkgo biloba à une concentration pondérale de 0,02 à 5 % (exprimée en matière sèche) et, de préférence de 0,05 % à 2 %. L'autre alpha-2-bloqueur est présent à une concentration de 0,01 à 15 % en poids. Lorsque cet alpha-2-bloqueur est l'escine, celui-ci est présent à raison de 0,05 à 4 % et, de préférence, de 0,1 à 2 %. Lorsque cet alpha-2-bloqueur est le lierre, celui-ci est présent à raison de 0,01 à 15 % et, de préférence, de 0,05 à 5 %.

Selon une forme de réalisation particulière de l'invention on peut adjoindre, à l'association selon l'invention d'autres composés lipolytiques comme ceux agissant au niveau de la phosphodiestérase, par exemple des bases xanthiques comme la caféine ou des β-agonistes tels que l'adrénaline, ses dérivés ou analogues ou d'autres composés lipolytiques.

Ces compositions peuvent se présenter sous différentes formes, en particulier sous forme anhydre telle que par exemple une huile ou un baume, ou encore d'émulsions H/E ou E/H ayant l'aspect d'une crème ou d'un lait ou sous forme de gels émulsionnés ou limpides, hydroalcooliques ou non, également sous forme de vésicules lipidiques et aussi sous forme de systèmes transdermiques.

Lorsque les compositions se présentent sous forme d'une émulsion, la phase grasse des émulsions représente de 10 à 80 % en poids, la phase aqueuse de 15 à 8 % en poids et l'agent émulsionnant de 2 à 30 % en poids par rapport au poids total de l'émulsion.

Parmi les agents émulsionnants, on utilise de préférence des polymères carboxyvinyliques de haut poids moléculaire, notamment le produit vendu sous le nom "Carbopol 941" par la Société GOODRICH, ainsi que des mélanges d'émulsionnants non ioniques, notamment le produit vendu sous la dénomination "Protegin X" vendu par la Société GOLDSCHMIDT.

Lorsque les compositions se présentent sous forme de vésicules, les principes actifs sont encapsulés dans des vésicules à base de lipides ioniques (liposomes) et ou de lipides non ioniques.

Les compositions selon l'invention peuvent également contenir divers ingrédients conventionnels tels que par exemple des tensio-actifs, des polymères, des huiles de silicone, des émollients, des épaississants, des parfums, des colorants, des édulcorants, des anti-oxydants ou des agents conservateurs. Comme cela a été dit précédemment, elles peuvent aussi contenir des agents lipolytiques connus comme la caféine.

Parmi les huiles de silicones, on utilise de préférence le cyclopentadimethylsiloxane, notamment le produit vendu sous le nom "Volatil Silicone 7158" par la Société Union Carbide ainsi que l'alkyldimethicone copolyol, notamment le produit vendu sous la dénomination "Abil We 09" par la Société GOLDSCHMIDT.

Parmi les tensioactifs, on utilise de préférence le monostéarate de glycérine, notamment le produit vendu sous le nom "Arlacel 165" par la Société ATLAS ainsi que le monostéarate de sorbitan oxyéthyléné (OE) à l'aide de 20 moles d'OE, notamment le produit vendu sous le nom de "Tween 60" par la Société ATLAS.

Parmi les agents émollients, on utilise de préférence les polymères d'oxyde d'éthylène, le cocoate de glycéryle oxyéthyléné à l'aide de 7 moles d'OE, notamment le produit vendu sous la dénomination "Cétiol HE" par la Société HENKEL, ainsi que le mélange de stéarate de glycol et de polyéthylène glycol (6 et 32 PEG), notamment le produit vendu sous la dénomination "Tefose 63" par la Société GATTEFOSSE.

Parmi les agents épaississants, on utilise de préférence les dérivés de silice tels que la silice colloïdale pyrogénée, notamment le produit vendu sous la dénomination "Aérosil 200" par la Société DEGUSSA AG, ainsi que l'acide polyacrylique réticulé, notamment le produit vendu sous la dénomination "Carbopol 940" par la Société GOODRICH.

La présente invention a en outre pour objet une méthode de traitement cosmétique, en vue d'améliorer l'aspect esthétique d'une personne, consistant à appliquer sur la peau une composition amincissante telle que définie ci-dessus. De façon générale, ce traitement est réalisé pendant une période de 6 à 8 semaines à raison d'une à deux applications par jour.

Les compositions cosmétiques amincissantes selon l'invention peuvent être également administrées par ionophorèse.

On va maintenant donner à titre d'illustration plusieurs exemples de compositions selon l'invention.

| EXEMPLE 1 : EMULSION H/E AMINCISSANTE | |
|---|---|
| Volatil Silicone 7158 | 10,0 g |
| Perhydrosqualène | 18,0 g |
| Huile de vaseline | 5,0 g |
| Lanoline liquide | 4,0 g |
| Arlacel 165 | 6,0 g |
| Tween 60 | 2,0 g |
| Alcool cétylique | 1,2 g |
| Acide stéarique | 2,5 g |
| Triéthanolamine | 0,1 g |
| Conservateur | 0,3 g |
| Antioxydants | 0,3 g |
| Caféine | 1,0 g |
| Extrait de ginkgo biloba (vendu par la Société BEAUFOUR) | 0,5 g |
| Propylène glycol | 5,0 g |
| Escine acide (vendu par la Société INVERNI) | 0,5 g |
| Hydroxyde de sodium | 0,008g |
| Eau déminéralisée qsp | 100,0 g |

| EXEMPLE 2 : EMULSION H/E AMINCISSANTE | |
|---|---|
| Propylène glycol | 2,0 g |
| PEG 400 | 3,0 g |
| Extrait de ginkgo biloba (vendu par la Société INVERNI) | 0,3 g |
| Caféine | 3,0 g |
| Benzoate de sodium | 3,0 g |
| Conservateur | 0,3 g |
| Parfum | 0,5 g |
| Extrait de lierre (vendu par la Société GATTEFOSSE) | 3,0 g |
| Carbopol 941 | 0,2 g |
| Myristate d'isopropyle | 1,0 g |
| Alcool cétylique | 3,0 g |
| Acide stéarique | 3,0 g |
| Monostéarate de glycérol | 3,0 g |
| Huile de germe de maïs | 2,0 g |
| Eau déminéralisée qsp | 100,0 g |

| EXEMPLE 3 : EMULSION E/H AMINCISSANTE | |
|---|---|
| Protegin X | 20,0 g |
| Huile de vaseline | 10,0 g |
| Composition aromatique | 1,0 g |
| Huile de tournesol | 15,0 g |
| Conservateur | 0,3 g |
| Glycérol | 5,0 g |
| Sulfate de magnésium | 0,5 g |
| Extrait de lierre (vendu par la Société INVERNI) | 0,5 g |
| Cétiol HE | 4,0 g |
| Extrait de ginkgo biloba (vendu par la Société INVERNI) | 1,0 g |
| Eau déminéralisée qsp | 100,0 g |

| EXEMPLE 4 : EMULSION E/H AMINCISSANTE | |
|---|---|
| Abil We 09 | 5,0 g |
| Myristate d'isopropyle | 5,0 g |
| Volatil silicone 7158 | 8,0 g |
| Huile de vaseline | 5,0 g |
| Aérosil 200 | 0,4 g |
| Huile de purcellin (vendue par la Société DRAGOCCO) | 14,0 g |
| Chlorure de sodium | 0,5 g |
| Transcutol | 3,0 g |
| Extrait de ginkgo biloba (vendu par la Société BEAUFOUR) | 0,5 g |
| Caféine | 1,0 g |
| Escine acide (vendu par la Société INVERNI) | 0,5 g |
| Hydroxyde de sodium | 0,008 g |
| Conservateur | 0,3 g |
| Eau déminéralisée qsp | 100,0 g |

| EXEMPLE 5 : GEL AMINCISSANT HYDROALCOOLIQUE | |
|---|---|
| Carbopol 940 | 0,9 g |
| Alcool éthylique | 20,0 g |
| Triéthanolamine | 0,3 g |
| Parfum | 0,3 g |
| Conservateur | 0,3 g |
| Transcutol | 5,0 g |
| Extrait de lierre (vendu par la Société INVERNI) | 1,0 g |
| Escine acide (vendu par la Société INVERNI) | 1,0 g |
| Extrait de Ginkgo biloba (vendu par la Société BEAUFOUR) | 0,05 g |
| Hydroxyde de sodium | 0,015 g |
| Eau déminéralisée qsp | 100,0 g |

| EXEMPLE 6 : GEL EMULSIONNE H/E AMINCISSANT | |
|---|---|
| Carbopol 940 | 0,6 g |
| Volatil Silicone 7158 | 3,0 g |
| Huile de purcellin (vendue par la Société DRAGOCCO) | 7,0 g |
| Tefose 63 | 3,0 g |
| Conservateur | 0,3 g |
| Parfum | 0,4 g |
| Alcool éthylique | 10,0 g |
| Triéthanolamine | 0,2 g |
| Caféine | 1,0 g |
| Cétiol HE | 2,0 g |
| Extrait de ginkgo biloba (vendu par la Société BEAUFOUR) | 0,8 g |
| Extrait de lierre (vendu par la Société INVERNI) | 0,5 g |
| Eau déminéralisée qsp | 100,0 g |

| EXEMPLE 7 : GEL AMINCISSANT | |
|---|---|
| Carbopol 940 | 0,6 g |
| Transcutol | 5,0 g |
| Triéthanolamine | 0,3 g |
| Conservateur | 0,3 g |
| Propylène glycol | 3,0 g |
| Extrait de lierre | 1,0 g |
| Escine acide | 0,5 g |
| Extrait de Ginkgo biloba (vendu par la Société INVERNI) | 5 g |
| Hydroxyde de sodium | 0,007 g |
| Caféine | 1,0 g |
| Eau déminéralisée qsp | 100,0 g |

| EXEMPLE 8 : CREME AMINCISSANTE AUX VESICULES NON IONIQUES | |
|---|---|
| Polyglyceryl-3 cétyl éther | 3,8 g |
| B-sitostérol | 3,8 g |
| Dicétyl-phosphate | 0,4 g |
| Conservateur | 0,3 g |
| Caféine | 1,5 g |
| Extrait de ginkgo biloba (vendu par la Société BEAUFOUR) | 0,3 g |
| Benzoate de sodium | 1,5 g |
| Transcutol | 3,0 g |
| Escine acide (vendu par la Société INVERNI) | 0,5 g |
| Hydroxyde de sodium | 0,007 g |
| Extrait de lierre (vendu par la Société GATTEFOSSE) | 3,0 g |
| Huile de tournesol | 35,0 g |
| Parfum | 0,6 g |
| Carbopol 940 | 0,2 g |
| Triéthanolamine | 0,2 g |
| Eau déminéralisée qsp | 100,0 g |

## Revendications

1. Utilisation du ginkgo biloba en tant qu'alpha-2-bloqueur en association avec au moins un autre alpha-2-bloqueur dans la préparation d'une composition amincissante destinée à une application topique.

2. Utilisation selon la revendication 1, caractérisée par le fait que la teneur totale en alpha-2-bloqueur dans la composition est supérieure à 0,03 % et de préférence comprise entre 0,05 et 20 % en poids.

3. Utilisation selon la revendication 1 ou 2, caractérisée par le fait que l'autre alpha-2-bloqueur est le lierre ou l'escine.

4. Utilisation selon l'une quelconque des revendications 1 à 3, caractérisée par le fait que le ginkgo biloba est présent à raison de 0,02 à 5 % en poids et de préférence de 0,05 à 2 %.

5. Utilisation selon l'une quelconque des revendications 1 à 4, caractérisée par le fait que le lierre est présent à raison de 0,01 à 15 % en poids et de préférence de 0,05 à 5 %.

6. Utilisation selon l'une quelconque des revendications 1 à 5, caractérisée par le fait que l'escine est présent à raison de 0,05 à 4 % en poids et de préférence de 0,1 à 2 %.

7. Utilisation selon l'une quelconque des revendications 1 à 6, caractérisée par le fait que la composition contient en outre au moins un agent lipolytique agissant au niveau de la phosphodiestérase tel que de la caféine.

8. Utilisation selon l'une quelconque des revendications 1 à 7, caractérisée par le fait que la composition contient en outre au moins un β-agoniste tel que l'adrénaline ou ses dérivés ou analogues.

9. Composition amincissante caractérisée par le fait qu'elle contient en association du ginkgo biloba en tant qu'alpha-2-bloqueur avec au moins un autre alpha-2-bloqueur.

10. Composition selon la revendication 9, caractérisée par le fait que la teneur totale en alpha-2-bloqueur dans la composition est supérieure à 0,03 % et de préférence comprise entre 0,05 et 20 % en poids.

11. Composition selon la revendication 9 ou 10, caractérisée par le fait que l'autre alpha-2-bloqueur est le lierre ou l'escine.

12. Composition selon l'une quelconque des revendications 9 à 11, caractérisée par le fait que le ginkgo biloba est présent à raison de 0,02 à 5 % en poids et de préférence de 0,05 à 2 %.

13. Composition selon l'une quelconque des revendications 9 à 12, caractérisée par le fait que le lierre est présent à raison de 0,01 à 15 % en poids et de préférence de 0,05 à 5 %.

14. Composition selon l'une quelconque des revendications 9 à 13, caractérisée par le fait que l'escine est présent à raison de 0,05 à 4 % et de préférence de 0,1 à 2 %.

15. Composition selon l'une quelconque des revendications 9 à 14, caractérisée par le fait qu'elle contient en outre au moins un agent lipolytique agissant au niveau de la phosphodiesterase tel que de la caféine.

16. Composition selon l'une quelconque des revendications 9 à 15, caractérisée par le fait qu'elle contient en outre au moins un β-agoniste tel que l'adrénaline ou ses dérivés ou analogues.

17. Méthode de traitement cosmétique en vue d'améliorer l'aspect esthétique d'une personne caractérisée par l'application sur la peau d'une composition amincissante selon l'une quelconque des revendications 9 à 16.

18. Méthode de traitement cosmétique selon la revendication 17, caractérisée par le fait qu'elle est associée à la diète et/ou au sport.

## Claims

1. Use of Ginkgo biloba as alpha-2-blocker in combination with at least one other alpha-2-blocker in the preparation of a slimming composition intended for topical application.

2. Use according to Claim 1, characterized by the fact that the total alpha-2-blocker content in the composition is greater than 0.03 % and preferably between 0.05 and 20 % by weight.

3. Use according to claim 1 or 2, characterized by the fact that the other alpha-2-blocker is ivy or escin.

4. Use according to any one of Claims 1 to 3, characterized by the fact that the Ginkgo biloba is present in an amount of 0.02 to 5 % by weight and preferably 0.05 to 2 %.

5. Use according to any one of Claims 1 to 4, characterized by the fact that the ivy is present in an amount of 0.01 to 15 % by weight and preferably 0.05 to 5 %.

6. Use according to any one of Claims 1 to 5, characterized by the fact that the escin is present in an amount of 0.05 to 4 % by weight and preferably 0.1 to 2 %.

7. Use according to any one of Claims 1 to 6, characterized by the fact that the composition contains, in addition, at least one lipolytic agent acting at the phosphodiesterase level such as caffeine.

8. Use according to any one of Claims 1 to 7, characterized by the fact that the composition contains, in addition, at least one β-agonist such as adrenaline or its derivatives or analogs.

9. Slimming composition characterized by the fact that it contains in combination Ginkgo biloba as alpha-2-blocker with at least one other alpha-2-blocker.

10. Composition according to Claim 9, characterized by the fact that the total alpha-2-blocker content in the composition is greater than 0.03 % and preferably between 0.05 and 20 % by weight.

11. Composition according to Claim 9 or 10, characterized by the fact that the other alpha-2-blocker is ivy or escin.

12. Composition according to any one of Claims 9 to 11, characterized by the fact that the Ginkgo biloba is present in an amount of 0.02 to 5 % by weight and preferably 0.05 to 2 %.

13. Composition according to any one of Claims 9 to 12, characterized by the fact that the ivy is present in an amount of 0.01 to 15 % by weight and preferably 0.05 to 5 %.

14. Composition according to any one of Claims 9 to 13, characterized by the fact that the escin is present in an amount of 0.05 to 4 % and preferably 0.1 to 2 %.

15. Composition according to any one of Claims 9 to 14, characterized by the fact that it contains, in addition, at least one lipolytic agent acting at the phosphodiesterase level such as caffeine.

16. Composition according to any one of Claims 9 to 15, characterized by the fact that it contains, in addition, at least one β-agonist such as adrenaline or its derivatives or analogs.

17. Method of cosmetic treatment for enhancing the aesthetic appearance of a person, characterized by the application to the skin of a slimming composition according to any one of Claims 9 to 16.

18. Method of cosmetic treatment according to Claim 17, characterized by the fact that it is combined with diet and/or sport.

## Patentansprüche

1. Verwendung von Ginkgo biloba als α₂-Blocker zusammen mit mindestens einem weiteren α₂-Blocker zur Herstellung einer für die topische Applikation bestimmten Schlankheitsmittelzusammensetzung.

2. Verwendung gemäß Anspruch 1, dadurch gekennzeichnet, daß der Gesamtgehalt an α₂-Blocker in der Zusammensetzung mehr als 0,03 Gew.-% und vorzugsweise zwischen 0,05 und 20 Gew.-% beträgt.

3. Verwendung gemäß Anspruch 1 oder 2, dadurch gekennzeichnet, daß der weitere α₂-Blocker Efeu oder Aescin darstellt.

4. Verwendung gemäß einem der Ansprüche 1 bis 3, dadurch gekennzeichnet, daß Ginkgo biloba in einer Menge von 0,02 bis 5 Gew.-% und vorzugsweise von 0,05 bis 2 Gew.-% vorliegt.

5. Verwendung gemäß einem der Ansprüche 1 bis 4, dadurch gekennzeichnet, daß der Efeu in einer Menge von 0,01 bis 15 Gew.-% und vorzugsweise von 0,05 bis 5 Gew.-% vorliegt.

6. Verwendung gemäß einem der Ansprüche 1 bis 5, dadurch gekennzeichnet, daß das Aescin in einer Menge von 0,05 bis 4 Gew.-% und vorzugsweise von 0,1 bis 2 Gew.-% vorliegt.

7. Verwendung gemäß einem der Ansprüche 1 bis 6, dadurch gekennzeichnet, daß das Mittel zusätzlich mindestens ein Lipolytikum wie Coffein enthält, das auf der Ebene der Phosphodiesterase eingreift.

8. Verwendung gemäß einem der Ansprüche 1 bis 7, dadurch gekennzeichnet, daß das Mittel zusätzlich noch mindestens einen β-Agonisten wie Adrenalin oder dessen Derivate oder Analoga enthält.

9. Schlankheitsmittel, dadurch gekennzeichnet, daß es eine Kombination aus Ginkgo biloba als α₂-Blocker mit mindestens einem weiteren α₂-Blocker enthält.

10. Zusammensetzung gemäß Anspruch 9, dadurch gekennzeichnet, daß der Gesamtgehalt an α₂-Blocker in der Zusammensetzung mehr als 0,03 Gew.-% und vorzugsweise zwischen 0,05 und 20 Gew.-% beträgt.

11. Zusammensetzung gemäß Anspruch 9 oder 10, dadurch gekennzeichnet, daß der weitere α₂-Blocker Efeu oder Aescin darstellt.

12. Zusammensetzung gemäß einem der Ansprüche 9 bis 11, dadurch gekennzeichnet, daß Ginkgo biloba in einer Menge von 0,02 bis 5 Gew.-% und vorzugsweise von 0,05 bis 2 Gew.-% vorliegt.

13. Zusammensetzung gemäß einem der Ansprüche 9 bis 12, dadurch gekennzeichnet, daß der Efeu in einer Menge von 0,01 bis 15 Gew.-% und vorzugsweise von 0,05 bis 5 Gew.-% vorliegt.

14. Zusammensetzung gemäß einem der Ansprüche 9 bis 13, dadurch gekennzeichnet, daß das Aescin in einer Menge von 0,05 bis 4 Gew.-% und vorzugsweise von 0,1 bis 2 Gew.-% vorliegt.

15. Zusammensetzung gemäß einem der Ansprüche 9 bis 14, dadurch gekennzeichnet, daß die Zusammensetzung zusätzlich mindestens ein Lipolytikum wie Coffein enthält, das auf der Ebene der Phosphodiesterase eingreift.

16. Zusammensetzung gemäß einem der Ansprüche 9 bis 15, dadurch gekennzeichnet, daß die Zusammensetzung zusätzlich noch mindestens einen β-Agonisten wie Adrenalin oder dessen Derivate oder Analoga enthält.

17. Verfahren zur kosmetischen Behandlung im Zusammenhang mit der Verbesserung des ästhetischen Erscheinungsbilds einer Person, dadurch gekennzeichnet, daß ein Schlankheitmittel gemäß einem der Ansprüche 9 bis 16 auf die Haut appliziert wird.

18. Verfahren zur kosmetischen Behandlung gemäß Anspruch 17, dadurch gekennzeichnet, daß es von Diät und/oder Sport begleitet wird.
